# EUROPEAN PATENT SPECIFICATION

(11) **EP 2 219 541 B1**
(45) Date of publication and mention of the grant of the patent: **05.09.2012**
(21) Application number: 08847796.3
(22) Date of filing: 05.11.2008
(51) Int. Cl.: A61B 17/88

(54) **AN INSTRUMENT FOR PLACING A "POLYAXIAL" BONE SCREW OF VERTEBRAL OSTEOSYNTHESIS EQUIPMENT**
INSTRUMENT ZUR PLATZIERUNG EINER POLYAXIALEN KNOCHENSCHRAUBE AUS DER VERTEBRALEN OSTEOSYNTHESE-AUSRÜSTUNG
INSTRUMENT POUR METTRE EN PLACE UNE VIS OSSEUSE POLY-AXIALE D'ÉQUIPEMENT D'OSTÉOSYNTHÈSE VERTÉBRALE

(30) Priority: 05.11.2007 FR 0707752
(43) Date of publication of application: 25.08.2010
(73) Proprietor: Medicrea International, 01700 Neyron (FR)
(72) Inventor: SOURNAC, Denys, F-01600 Reyrieux (FR); RYAN, David, F-69660 Collonges Au MontD'or (FR)
(74) Representative: Jeannet, Olivier
(86) International application number: PCT/IB2008/054606
(87) International publication number: WO 2009/060389

(56) References cited:
- EP-A- 0 880 943
- EP-A- 1 092 510
- WO-A-93/21848
- JP-A- 2003 265 492

## Description

The present invention relates to an instrument for placing a bone screw, notably a so-called "polyaxial" screw of vertebral osteosynthesis equipment.

A piece of vertebral osteosynthesis equipment frequently comprises fixing screws, each of which includes a distal threaded portion, intended to be inserted in a vertebra generally at a pedicle, and a proximal threaded portion, used for mounting the piece of equipment on the vertebra. This proximal threaded portion is frequently jointed relatively to the distal threaded portion, the screw being then currently designated as "polyaxial" screw.

Such a polyaxial screw is set into place by means of an instrument comprising an internal tube, an external tube and means for driving the internal tube into rotation relatively to the external tube. The internal tube comprises a tapped distal end, intended to be engaged with the proximal threaded portion of the screw, and the external tube comprises a distal end intended to be engaged with a shoulder formed by the distal threaded portion of the screw; the rotational driving means comprise a knurl mounted on the internal tube. In practice, the distal tapped end of the internal tube is brought into engagement with the proximal threaded portion of the screw and then the knurl is actuated in rotation in the direction for screwing the internal tube on the threaded proximal end so as to bring the shoulder formed by the distal threaded portion of the screw into engagement with the distal end of the external tube. The thereby generated axial tension on the screw allows the instrument to perfectly grasp this screw, with blocking of the screw in rotation relatively to the instrument. The latter may then be used for placing the screw in the pedicle of a vertebra, without the proximal threaded portion of the screw being an obstacle to its placement.

With the existing instrument, the significant drawback of rotational blocking of the internal tube-knurl assembly after placement of the screw is reported, frequently requiring that the practitioner use a key for untightening this assembly in order to release the screw after placement. It is difficult for the practitioner to determine a tightening of the knurl both sufficient for ensuring perfect connection of the screw to the instrument and insufficient for achieving blocking of the knurl.

Document EP 0880 943 A1 discloses an instrument according to the preamble of claim 1.

The main object of the present invention is to find a remedy to this drawback.

Another object of the invention is to achieve this main object with an instrument structure which remains easily sterilizable without any substantial increase in the manufacturing cost of the instrument.

The relevant instrument comprises in a way known *per se,* a first component intended to engage with the threaded proximal portion of the screw, a second component intended to engage with a head of the distal threaded portion of the screw, and means for driving the first component into rotation relatively to the second component, so that, in a direction of rotation corresponding to the mounting of the screw on the instrument, said first component may be brought so as to engage with the threaded proximal portion of the screw, and said head of the distal threaded portion of the screw to engage with said second component, these driving means comprising an actuation member for performing said driving into rotation.

According to the invention, said actuation member is rotationally bound to said first component via releasable connection means, being released beyond torque threshold exerted on this actuation member in the direction of rotation corresponding to the mounting of the screw on the instrument.

With the instrument according to the invention, it is thereby possible to avoid that a tightening torque be exerted on the actuation member which may lead after placement of the screw to an impossibility of manually untightening this actuation member.

Preferably, the releasable connection means comprise:
- a first series of teeth laid out on said first component or on a part integral with this first component;
- a second series of teeth laid out on the actuation member, and
- holding means capable of maintaining the teeth of both of these series of teeth, in engagement with each other until said tightening torque is reached and allowing these teeth to be disengaged when the tightening torque is reached.

Said maintaining means may notably comprise an elastic member normally maintaining the teeth engaged with each other and the elastic deformation of which allows the teeth to become disengaged.

Preferably,
- said first series of teeth is laid out in the proximal end of said first component or on a part integral with this first component;
- said actuation member has the shape of a bushing which will cap the proximal end of the first component or of said part integral with this first component, comprising a bottom in which said second series of teeth is laid out, and
- said proximal end or said part and said bushing form respective abutment surfaces between which a spring is interposed, forming said maintaining means.

According to a preferred embodiment of the invention, in this case:
- said part forms a shoulder forming a first abutment surface;
- the actuation member comprises an external part including said bottom and comprising a tapped peripheral wall and an inner part comprising a threaded peripheral wall and an internal shoulder forming a second abutment surface.

The actuation member is thus made up by engaging said inner part onto the proximal end of the first component, by placing the spring between the respective abutment surfaces, and then by screwing said external part onto said inner part.

Said inner part may form a distal housing receiving an O-ring gasket opposing the inflow of fluids into the interior of the actuation member.

The housing receiving the spring is thus protected with regard to penetration of such fluids.

Said first component advantageously comprises a distal tube, the distal end of which is tapped in order to engage with the proximal threaded portion of the screw, this tube being connected on the proximal side, to a proximal part, coaxial therewith, comprising said first series of teeth laid out in its proximal end.

Said second component advantageously comprises a distal tubular portion in which said first component is engaged and a cage-shaped proximal portion delimiting a housing in which said actuation member is found.

The invention will be well understood, and other features and advantages thereof will become apparent with reference to the appended schematic drawing, illustrating as a non-limiting example, a preferred embodiment of the instrument to which it relates.

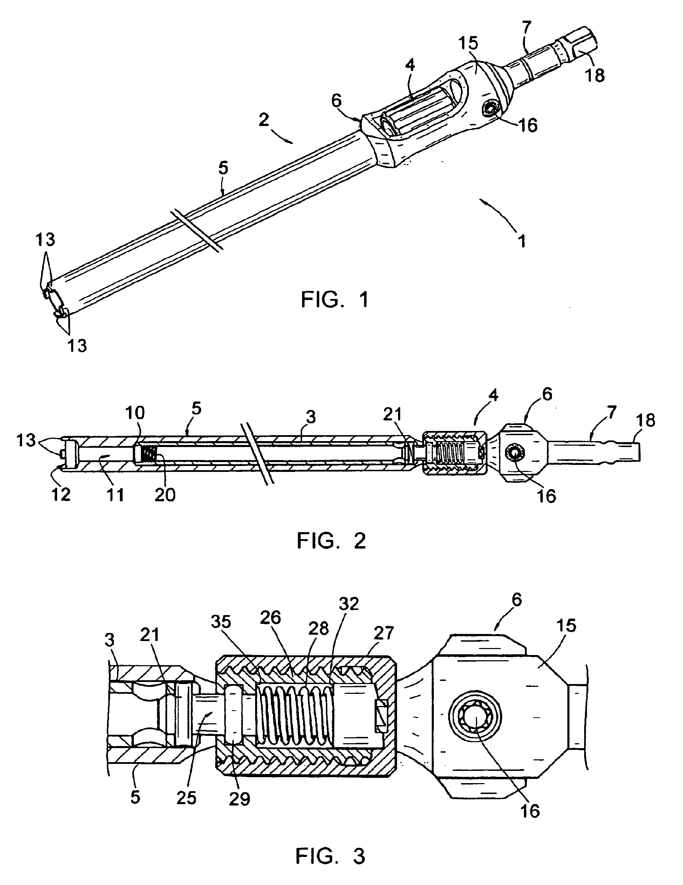

Fig. 1 is a perspective view thereof;
Fig. 2 is a longitudinal sectional view thereof;
Fig. 3 is view thereof, similar to Fig. 2, at an enlarged scale;
Fig. 4 is an exploded perspective view thereof;
Fig. 5 is a perspective view of a proximal part of a first component which it comprises, and
Fig. 6 is a perspective view of an actuation member which it comprises.

The figures illustrate an instrument 1 for placing a so-called "polyaxial" bone screw of vertebral osteosynthesis equipment.

This screw is as described in French Patent Application No. 07 07468. It includes a distal threaded portion and a proximal threaded pin jointed relatively to the distal threaded portion.

The latter comprises a threaded body and a proximal head. The threaded body is intended to be inserted into a vertebra, generally at a pedicle. The head forms a proximal cavity intended to receive with jointing, the proximal threaded pin and comprises a proximal collar in which four radial notches are laid out, positioned at 90 degrees from each other.

The proximal threaded pin comprises a threaded rod with which it may be assembled with other parts which the piece of equipment comprises (in particular to a stirrup for connecting to a longitudinal linking rod) and a distal bulging portion intended to be received with jointing, in said proximal cavity of the distal threaded portion.

This distal bulging portion and this proximal cavity at their periphery have locking shapes so that the pins may be locked in rotation relatively to the distal threaded portion while allowing the pin to be jointed relatively to this distal threaded portion. These locking shapes may notably be hexagonal with rounded edges as described in French Patent Application No. 07 07468.

As this is illustrated in the figures of the present patent application, the instrument 1 comprises an external body 2, an internal tube 3 and an assembly 4 for maneuvering the internal tube 3 relatively to the external body 2.

The external body 2 comprises a distal tubular portion 5, an intermediate portion 6 and a proximal portion 7.

The distal tubular portion 5 contains the internal tube 3, which is engaged into it in an adjusted way with possibility of rotation. It forms a distal shoulder 10, against which the distal end of the internal tube 3 is intended to abut. It also forms a distal bore 11 and, at its distal end, a cavity 12 and four teeth 13 positioned at 90 degrees from each other. The distal bore 11 is intended to receive the proximal threaded pin of the screw, which may engage by screwing with a tapped thread 20 positioned in the distal end of the internal tube 3; the cavity 12 as for it receives the head of the distal threaded portion of the screw until the distal end of the distal tubular portion 5 bears against the collar of the screw. During this bearing action, the teeth 13 will engage into the notches which this collar comprises, achieving engagement of the screw and of the instrument 1.

The intermediate portion 6 forms a body with the distal tubular portion 5 and is cage-shaped, delimiting a housing for receiving the maneuvering assembly 4. On the proximal side, the intermediate portion 6 comprises an end 15 pierced with an axial bore, for the engagement of the internal tube 3 and of the maneuvering assembly 4 through it, and with a transverse bore receiving a screw spindle 16 for mounting the proximal portion 7.

The proximal portion 7 comprises a proximal square-shaped end 18, allowing the instrument 1 to be maneuvered in rotation for screwing or unscrewing the screw, and a distal portion pierced with a transverse bore for receiving the spindle 16.

The internal tube 3 comprises, in addition to the tapped thread 20, a transverse bore laid out in its proximal end, intended to receive a transverse spindle 21 for rotatably connecting this tube 3 to the maneuvering assembly 4.

The maneuvering assembly 4 has an internal rod 25, a threaded internal part 26, a tapped external part 27, a spring 28 and a seal gasket 29.

The internal rod 25 comprises a cylindrical body 30 and a head 31. The distal end of the body 30 comprises a through-hole intended to be crossed by the spindle 21 in order to allow the rotary connection of the part 25 and of the internal tube 3. The head 31 has a widened diameter relatively to the diameter of the body 30 so that it delimits with the latter a shoulder 32 turned towards the distal side.

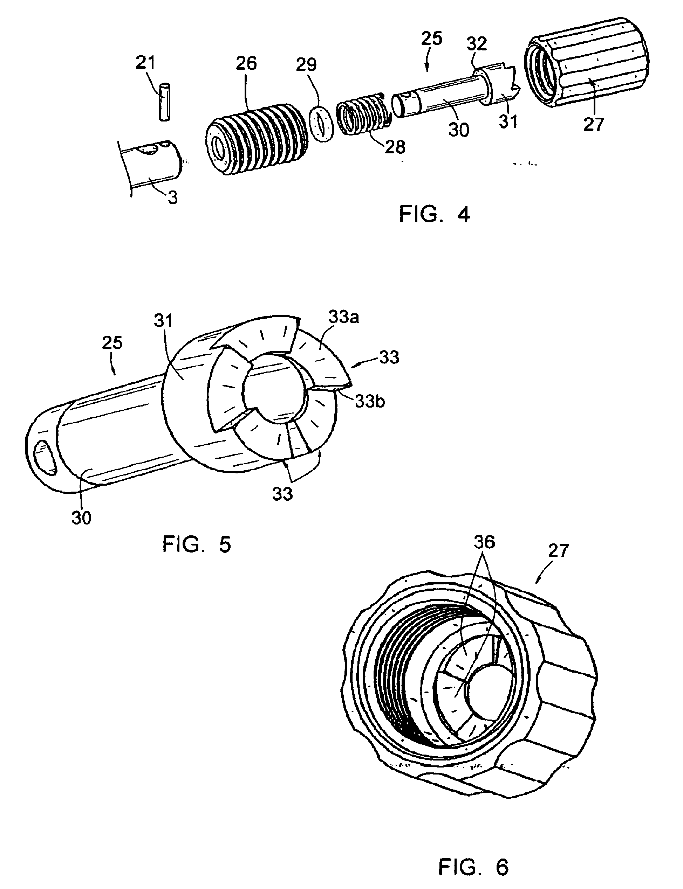

At its proximal end, this head 31 comprises, as this is more particularly shown in Fig. 4, a series of five teeth 33 laid out on the whole of its periphery, each tooth 33 comprising a tilted flank 33a forming the proximal end of the head 31, and a straight flank 33b, positioned parallel to the longitudinal axis of the part 25. The tilt of each tilted flank 33a relative to this longitudinal axis is such that it forms an angle of the order of 70-80 degrees with this axis.

The threaded internal part 26 comprises a distal axial bore and a proximal axial bore, the distal bore being intended to receive in an adjusted way, the body 30 of the internal rod 25, while the proximal bore is intended to receive in an adjusted way the head 31 of this same rod 25. The part 26 thereby forms a shoulder 35 turned towards the proximal side. At its wall delimiting the distal bore, the part 26 comprises a groove for receiving the seal gasket 29.

The tapped external part 27 has the shape of a bushing, i.e. it comprises a tapped peripheral wall, allowing it to be screwed onto the threaded internal part 26, and a bottom. This bottom comprises, as this is most particularly visible in Fig. 5, a series of teeth 36 of the same shape as the teeth 33, able to cooperate with the latter in order to form releasable connecting means, the operation of which will be explained later on.

The outer face of the tapped external part 27 is ribbed, so as to promote its gripping by hand for exerting a tightening or untightening torque on this part.

The spring 28 is interposed between the shoulders 32 and 35 and allows the head 31 to be pressed against the bottom of the tapped external part 27 so that the teeth 33 are normally maintained engaged with the teeth 36 and so there exists a rotary connection between the part 27 and the internal tube 3.

In practice, the pin of the screw is inserted into the bore 11 and the tube 3 is screwed onto it by rotationally actuating the part 27, until the head which this screw comprises, is introduced into the cavity 12 and the collar which this screw also comprises, abuts against the distal end of the distal tubular portion 6. When this abutment is achieved, possible continuation of the manoeuvre in rotation of the part 27 in the tightening direction will have the effect of sliding the tilted flanks of the teeth 36 against the corresponding tilted flanks 33a of the teeth 33, causing an axial displacement of the part 26-part 27 assembly relatively to the rod 25 against the elastic force of the spring 28, until these teeth 36, 33 escape. The rotary connection of the part 27 relatively to the tube 3 is thus released beyond a torque threshold exerted on the part 27 in the direction of rotation corresponding to the mounting of the screw on the instrument 1, by this release it is possible to prevent a tightening torque from being exerted on the part 27, which may lead after placement of the screw, to an impossibility of manually unscrewing this part 27.

When the part 27 is manoeuvred in the unscrewing direction, the straight flanks of the teeth 26 bear against the straight flanks 33b of the teeth 33 and ensures the unscrewing of the internal tube 3 relatively to the proximal pin of the screw.

As this appears in the foregoing, the invention provides an instrument for placing a bone screw, notably a so-called "polyaxial" screw of vertebral osteosynthesis equipment, having determining advantages as compared with homologous instruments of the prior art.

The invention was described above with reference to an embodiment described as a pure example. It is obvious that it is not limited to this embodiment but that it extends to all embodiments covered by the appended claims herein.

## Claims

1. An instrument (1) for placing a bone screw including a distal threaded portion, notably a so called "polyaxial" bone screw of vertebral osteosynthesis equipment, this bone screw comprising a distal threaded portion having a head and a proximal threaded portion; the instrument (1) comprises a first component (3) intended to engage with the threaded proximal portion of the screw, a second component (2) intended to engage with said head of the distal threaded portion of the screw, and means (4) for driving the first component (3) into rotation relatively to the second component (2), so that, in a direction of rotation corresponding to the mounting of the screw on the instrument (1), said first component (3) may be brought so as to engage with the threaded proximal portion of the screw, and said head of said distal threaded portion to engage with said second component (2), these driving means (4) comprising an actuation member (26, 27) for performing said driving into rotation;
**characterized in that** said actuation member (26, 27) is rotationally bound to said first component (3) via releasable connection means (33, 36), being released beyond a torque threshold exerted on this actuation member (26, 27) in the direction of rotation corresponding to the mounting of the screw on the instrument (1).

2. An instrument (1) according to claim 1, caracterized in that the releasable connection means comprise:
- a first series of teeth (33) laid out on said first component (3) or on a part (25) integral with this first component (3);
- a second series of teeth (36) laid out on the actuation member (26, 27), and
- holding means (28) capable of maintaining the teeth (33, 36) of both of these series of teeth in engagement with each other until said torque threshold is reached and allowing these teeth to be disengaged when the tightening torque is reached.

3. An instrument (1) according to claim 2, caracterized in that said maintaining means comprise an elastic member (28) normally maintaining the teeth (33, 36) engaged with each other and the elastic deformation of which allows the teeth to become disengaged.

4. An instrument (1) according to claim 2 or claim 3, caracterized in that:
- said first series of teeth (33) is laid out in the proximal end of said first component (3) or on a part (25) integral with this first component;
- said actuation member (26, 27) has the shape of a bushing which will cap the proximal end of the first component (3) or of said part (25) integral with this first component, comprising a bottom in which said second series of teeth (36) is laid out, and
- said proximal end or said part (25) and said bushing form respective abutment surfaces (32, 35) between which a spring (28) is interposed, forming said maintaining means.

5. An instrument (1) according to claim 4, caracterized in that:
- said part (25) forms a shoulder (22) forming a first abutment surface;
- the actuation member (26, 27) comprises an external part (27) including said bottom and comprising a tapped peripheral wall and an inner part (26) comprising a threaded peripheral wall and an internal shoulder (35) forming a second abutment surface.

6. An instrument (1) according to claim 5, caracterized in that said inner part (26) forms a distal housing receiving an O-ring gasket (29) opposing the inflow of fluids into the interior of the actuation member (26, 27).

7. An instrument (1) according to any of claims 1 to 6, caracterized in that said first component (3) comprises a distal tube, the distal end of which is tapped in order to engage with the proximal threaded portion of the screw, this tube being connected on the proximal side, to a proximal part (25), coaxial therewith, comprising said first series of teeth (33) laid out in its proximal end.

8. An instrument (1) according to any of claims 1 to 7, caracterized in that said second component (2) comprises a distal tubular portion (5) in which said first component (3) is engaged and a cage-shaped proximal portion (6) delimiting a housing in which said actuation member (26, 27) is found.

## Patentansprüche

1. Instrument (1) zur Platzierung einer Knochenschraube einer Vorrichtung zur vertebralen Osteosynthese, wobei diese Knochenschraube einen distalen Gewindeabschnitt mit einem Kopf und einen proximalen Gewindeabschnitt umfasst; wobei das Instrument (1) eine erste Komponente (3), die zum Eingriff mit dem proximalen Gewindeabschnitt der Schraube bestimmt ist, eine zweite Komponente (2), die zum Eingriff mit dem Kopf des distalen Gewindeabschnitts der Schraube bestimmt ist, und Antriebsmittel (4) umfasst, um die erste Komponente (3) in Bezug auf die zweite Komponente (2) in Drehung zu versetzen, so dass in eine Drehrichtung entsprechend der Befestigung der Schraube am Instrument (1) die erste Komponente (3) dazu gebracht werden kann, in den proximalen Gewindeabschnitt der Schraube einzugreifen, und der Kopf des distalen Gewindeabschnitts dazu gebracht werden kann, in die zweiten Komponente (2) einzugreifen, wobei diese Antriebsmittel (4) ein Betätigungselement (26, 27) zum Ausführen der Drehung umfassen;
**dadurch gekennzeichnet, dass** das Betätigungselement (26, 27) mit der ersten Komponente (3) über lösbare Verbindungsmittel (33, 36) rotatorisch verbunden ist, wobei diese über einer Drehmomentgrenze gelöst werden, die auf dieses Betätigungselement (26, 27) in der Drehrichtung entsprechend der Befestigung der Schraube am Instrument (1) ausgeübt wird.

2. Instrument (1) nach Anspruch 1, **dadurch gekennzeichnet, dass** die lösbaren Verbindungsmittel Folgendes umfassen:
- eine erste Reihe von Zähnen (33), die auf der ersten Komponente (3) oder auf einem Bauteil (25), das mit der ersten Komponente (3) fest verbunden ist, angeordnet sind;
- eine zweite Reihe von Zähnen (36), die auf dem Betätigungselement (26, 27) angeordnet sind, und
- Haltemittel (28), das in der Lage ist, die Zähne (33, 36) beider Reihen von Zähnen in gegenseitigem Eingriff zu halten, bis die Drehmomentgrenze erreicht ist, und es ermöglicht, den Eingriff der Zähne zu lösen, wenn das Anziehdrehmoment erreicht ist.

3. Instrument (1) nach Anspruch 2, **dadurch gekennzeichnet, dass** das Festhaltemittel ein elastisches Element (28) umfasst, das die Zähne (33, 36) normalerweise in gegenseitigem Eingriff festhält und dessen elastische Verformung es ermöglicht, den Eingriff der Zähne zu lösen.

4. Instrument (1) nach Anspruch 2 oder Anspruch 3, **dadurch gekennzeichnet, dass**:
- die erste Reihe von Zähnen (33) im proximalen Ende der ersten Komponente (3) oder auf einem Bauteil (25), das mit der ersten Komponente fest verbunden ist, angeordnet ist;
- das Betätigungselement (26, 27) die Form einer Buchse aufweist, die das proximale Ende der ersten Komponente (3) oder des Bauteils (25), das mit der ersten Komponente fest verbunden ist, abdeckt, umfassend einen Boden, in dem die zweite Reihe von Zähnen (36) angeordnet ist, und
- das proximale Ende oder das Bauteil (25) und die Buchse entsprechende Anschlagflächen (32, 35) ausbilden, zwischen denen eine als Feder (28) ausgebildetes Festhaltemittel eingefügt ist.

5. Instrument (1) nach Anspruch 4, **dadurch gekennzeichnet, dass**:
- das Bauteil (25) eine Schulter (22) ausbildet, die eine erste Anschlagfläche ausbildet;
- das Betätigungselement (26, 27) ein äußeres Bauteil (27), das den Boden und eine umlaufende Wand mit Innengewinde umfasst, und ein inneres Bauteil (26) umfasst, das eine umlaufende Wand mit Außengewinde und eine innere Schulter (35) umfasst, die eine zweite Anschlagfläche ausbildet.

6. Instrument (1) nach Anspruch 5, **dadurch gekennzeichnet, dass** das innere Bauteil (26) ein distales Gehäuse bildet, das eine O-RingDichtung (29) aufnimmt, die dem Eindringen von Flüssigkeiten in das Innere des Betätigungselements (26, 27) entgegen wirkt.

7. Instrument (1) nach einem der Ansprüche 1 bis 6, **dadurch gekennzeichnet, dass** die erste Komponente (3) ein distales Rohr umfasst, dessen distales Ende mit einem Innengewinde zum Eingriff in den proximalen Gewindeabschnitt der Schraube versehen ist, wobei dieses Rohr auf der proximalen Seite mit einem proximalen Bauteil (25) verbunden ist, das koaxial dazu verläuft und die erste Reihe von Zähnen (33) umfasst, die in seinem proximalen Ende angeordnet sind.

8. Instrument (1) nach einem der Ansprüche 1 bis 7, **dadurch gekennzeichnet, dass** die zweite Komponente (2) einen distalen Rohrabschnitt (5), in den die erste Komponente (3) eingreift, und einen gehäuseförmigen proximalen Abschnitt (6) umfasst, der ein Gehäuse begrenzt, in dem sich das Betätigungselement (26, 27) befindet.

## Revendications

1. Instrument (1) de pose d'une vis osseuse d'un matériel d'ostéosynthèse vertébrale, cette vis à os comprenant une portion filetée distale ayant une tête et une portion filetée proximale ; l'instrument (1) comprend un premier élément (3) destiné à venir en prise avec la portion proximale filetée de la vis, un deuxième élément (2) destiné à venir en engagement avec ladite tête de la portion distale filetée de la vis, et des moyens (4) d'entraînement en rotation du premier élément (3) par rapport au deuxième élément (2), permettant, dans un sens de rotation correspondant au montage de la vis sur l'instrument (1), d'amener ledit premier élément (3) en prise avec la portion proximale filetée de la vis, et d'amener ladite tête de la portion distale filetée en prise avec ledit deuxième élément (2), ces moyens d'entraînement (4) comprenant un organe d'actionnement (26, 27) pour opérer ledit entraînement en rotation ;
**caractérisé en ce que** ledit organe d'actionnement (26, 27) est lié en rotation audit premier élément (3) par l'intermédiaire de moyens de liaison libérables (33, 36), se libérant au delà d'un seuil de couple exercé sur cet organe d'actionnement (26, 27) dans le sens de rotation correspondant au montage de la vis sur l'instrument (1).

2. Instrument (1) selon la revendication 1, **caractérisé en ce que** les moyens de liaison libérables comprennent :
- une première série de dents (33) aménagée sur ledit premier élément (3) ou sur une pièce (25) solidaire de ce premier élément (3) ;
- une deuxième série de dents (36) aménagée sur l'organe d'actionnement (26, 27), et
- des moyens de maintien (28) propres à maintenir les dents (33, 36) de ces deux séries de dents en prise mutuelle jusqu'à ce que soit atteint ledit seuil de couple de serrage et à permettre la venue de ces dents hors de prise lorsque ce couple de serrage est atteint.

3. Instrument (1) selon la revendication 2, **caractérisé en ce que** lesdits moyens de maintien comprennent un organe élastique (28) maintenant normalement les dents (33, 36) en prise les unes avec les autres et dont la déformation élastique permet la venue de ces dents hors de prise.

4. Instrument (1) selon la revendication 2 ou la revendication 3, **caractérisé en ce que** :
- ladite première série de dents (33) est aménagée dans l'extrémité proximale dudit premier élément (3) ou sur une pièce (25) solidaire de ce premier élément ;
- ledit organe d'actionnement (26, 27) présente la forme d'une douille venant coiffer l'extrémité proximale du premier élément (3) ou de ladite pièce (25) solidaire de ce premier élément, comprenant un fond dans lequel est aménagée ladite deuxième série de dents (36), et
- ladite extrémité proximale ou ladite pièce (25) et ladite douille forment des surfaces de butée respectives (32, 35) entre lesquelles est interposé un ressort (28) formant lesdits moyen de maintien.

5. Instrument (1) selon la revendication 4, **caractérisé en ce que** :
- ladite pièce (25) forme un épaulement (32) constituant une première surface de butée ;
- l'organe d'actionnement (26, 27) comprend une pièce externe (27) incluant ledit fond et comprenant une paroi périphérique taraudée, et une pièce intérieure (26) comprenant une paroi périphérique filetée et un épaulement interne (35) formant une deuxième surface de butée.

6. Instrument (1) selon la revendication 5, **caractérisé en ce que** ladite pièce intérieure (26) forme un logement distal recevant un joint torique (29) s'opposant à l'entrée de fluides à l'intérieur de l'organe d'actionnement (26, 27).

7. Instrument (1) selon l'une des revendications 1 à 6, **caractérisé en ce que** ledit premier élément (3) comprend un tube distal dont l'extrémité distale est taraudée pour venir en prise avec la portion filetée proximale de la vis, ce tube étant relié, du côté proximal, à une pièce proximale (25) coaxiale à lui, comprenant ladite première série de dents (33) aménagée dans son extrémité proximale.

8. Instrument (1) selon l'une des revendications 1 à 7, **caractérisé en ce que** ledit deuxième élément (2) comprend avantageusement une partie tubulaire distale (5) dans lequel est engagé ledit premier élément (3) et une partie proximale (6) en forme de cage, délimitant un logement dans lequel se trouve ledit organe d'actionnement (26, 27).
